Europäisches Patentamt

European Patent Office

Office européen des brevets

(11) Publication number: **0 127 327**
**A1**

# EUROPEAN PATENT APPLICATION

(21) Application number: 84302865.5

(22) Date of filing: 27.04.84

(51) Int. Cl.³: **C 12 Q 1/68**

(30) Priority: 29.04.83 GB 8311905

(43) Date of publication of application:
05.12.84 Bulletin 84/49

(84) Designated Contracting States:
CH DE FR GB IT LI NL

(71) Applicant: NATIONAL RESEARCH DEVELOPMENT CORPORATION
101 Newington Causeway
London SE1 6BU(GB)

(72) Inventor: Cox, Robert Ashley
Belstone 6 Gills Hill Lane
Radlett Hertfordshire WD7 8DF(GB)

(72) Inventor: Smulian, Nadia Jane
2 Staleys Road Borough Green
Sevenoaks Kent TN15 8RR(GB)

(74) Representative: Percy, Richard Keith
Patent Department National Research Development
Corporation 101 Newington Causeway
London SE1 6BU(GB)

(54) Method of determining nucleotide sequences in cells and of isolating nucleic acids from cells.

(57) It has been a problem that nucleic acids present in cells have had to be isolated by difficult extraction procedures, using e.g. phenol or a protease, before they can be assayed.

The present invention provides an assay method which can be carried out on crude cell lysate, without the need for any such extraction.

The method comprises lysing the cells in a lysis buffer comprising a chaotropic agent for disrupting nucleoproteins and which inhibits the nuclease, e.g. a guanidinium salt present in the lysate in a concentration of at leat 3 M at the time of hybridisation, when DNA is being determined treating the lysate to single-strand it, contacting the lysate under hybridisation conditions with a polynucleotide having a nucleotide sequence complementary to a sequence present in the nucleic acid to be determined, and determining the extent of hybridisation, e.g. by a sandwich or competition assay.

A similar procedure can be used to extract the nucleic acid from the cell by a hybridisation method and isolate the desired nucleic acid from the resultant hybrid.

EP 0 127 327 A1

- 1 -

127033

## METHOD OF DETERMINING NUCLEOTIDE SEQUENCES IN CELLS
## AND OF ISOLATING NUCLEIC ACIDS FROM CELLS

This invention relates primarily to the determination or assay of nucleotide sequences, including DNA and RNA sequences, present in cells. The terms "determination" and "assay" are used herein to cover qualitative detection of the presence or absence and the quantitative or semi-quantitative measurement of the amount of the nucleotide sequences. A secondary aspect of the invention relates to isolating polynucleotides, in practice nucleic acids, from cells.

The determination of nucleotide sequences in cells, e.g. for identification of a virus or bacterium, has conventionally required the isolation of nucleic acid from cells. The isolated nucleic acid can then be determined by hybridisation to a polynucleotide having an appropriate complementary sequence. RNA is normally present in cells mainly in a single-stranded form. DNA is normally present in the bihelical (double-stranded) form but can readily be made single-stranded by heating or the action of alkali. In the hybridisation assay, the polynucleotide is conveniently insolublised, e.g. by bonding to a support, and the amount of nucleic acid in the solid phase determined by labelling, in a manner analogous to immunoassay.

The isolation of nucleic acid from cells is made necessary mainly because of the presence in the cells of nucleases, i.e. enzymes which break down the RNA or DNA. These nucleases have to be inactivated or removed, since otherwise the nucleic acid will break down rapidly and so give a false result in the hybridisation assay.

Inactivation of nucleases in cell lysates is possible. For example detergents have some inhibitory effect on some nucleases. A better class of inactivators are the so-called chaotropes. These agents disrupt the cellular nucleoproteins in which nucleic acid is complexed to ribosomal protein. They are thought to act by causing chaos in the ordered hydrogen bonding structure of water in the vicinity of the nucleoprotein and thereby causing conformational change to the protein, resulting in it taking up a conformation which is less thermally stable to hydrolysis than the natural form. These chaotropes also inhibit nucleases. They have been described for use in extracting RNA from tissue cells, see, for example, R.A. Cox in "Methods in Enzymology", Volume 12B, 1968, pages 120-129, J.D. Harding and W.J. Rutter, J. Biol. Chem. 253, 8736-8740 (1978) and T. Maniatis et al., "Molecular Cloning: a laboratory manual", Cold Spring Harbor Laboratory, New York 1982, pages 189-190. Typical such chaotropes are guanidinium chloride and isothiocyante. They are used in high molar concentrations in order to be effective. High molar concentrations of salts have long been avoided in the choice of solvents promoting hybridisation (possibly because they cause elevation of the temperature at which hybridisation takes place, resulting in problems of thermal instability of the reactants), A typical salt concentration employed in hybridisation is 0.3 to 0.9 M. The idea of inactivating the nuclease in cell lysates in a high salt lysis buffer and probing the crude lysates directly with a complementary polynucleotide was unattractive.

The removal of nucleases from cell lysates has been carried out by various extraction procedures. In one popular procedure phenol is used as an extractant. In phenol extraction some nucleases are removed into the phenol phase and some are denatured. Other proteins are denatured and appear as a precipitate at the phenol/water interface. Chloroform, or a mixture thereof with another organic liquid, can be used as an alternative to phenol.

Another method of extraction employs a protease, e.g. proteinase K. These enzymes digests proteins including nucleases but do so only slowly. To inhibit nucleases while they are awaiting digestion, a detergent such as SDS is used.

All these extraction procedures are conventionally used to purify the nucleic acid so that it is free of cellular components including nucleases and nucleoproteins which would interfere with a successful hybridisation assay. They are, however, very time-consuming.

It has now surprisingly been found, despite the prejudice in the art against performing hybridisations in solutions of high molar concentrations of salts, that it is in fact possible to perform a successful hybridisation assay on a cell lysate containing a chaotrope, as described above. This surprising finding came about while the inventors were investigating slime moulds, from which they wished to isolate RNA free of DNA, using hybridisation for this isolation. They tried this procedure because time was of the essence. As it happened, by coincidence, slime moulds are particularly appropriate organisms to use because their high nuclease content makes conventional extraction procedures particularly difficult.

The invention has solved an awkward problem by making it possible, it is believed for the first time, to carry out a hybridisation assay directly on a crude cell lysate, without the need to perform a solvent extraction or protease digest.

The nearest prior art known to us consists of two literature papers, describing procedures out of the ordinary run. One, by U. Lindberg and B. Sundquist, J. Mol. Biol., 86, 451-469 (1974) describes the isolation of mRNA from polysomes. Polysomes (polyribosomes) are particles of large molecular weight consisting of several ribosomes attached to the same mRNA which are involved in the synthesis of proteins from the RNA within the cell. These authors used EDTA to disrupt the polysomes into ribosomal sub-particles and thereby release the mRNA from the complex, leaving nucleoproteins containing ribosomal RNA and proteins. This method is only of interest where mRNA is desired. They then found it possible to perform an affinity chromatography using oligo-dT cellulose to interact with the mRNA which had a poly-A tail. Since these workers started with polysomes, rather than whole cells, and since they used an EDTA extraction initially, the present invention is clearly distinguished over this paper.

The second reference is P.L. Collins, L.E. Hightower and L.A. Ball, Journal of Virology 324-336 (1978) and relates to preparation of Newcastle Disease Virus RNA from chicken cells. In one procedure the lysate was incubated with proteinase K and the RNA extracted with a mixture of phenol, chloroform and amyl alcohol. In an alternative, cells were lysed on a plate in cold buffer containing two detergents. The lysate was separated from nuclei adhering to the plate. The lysate was then treated with 0.4 M-NaCl and 0.5% SDS and the RNA was isolated by oligo-dT cellulose affinity chromatography, again taking adantage of the poly-A tail on the RNA. It is unclear from the paper how successful this procedure proved to be, since the results of translation from the RNA isolated do not distinguish between the two procedures in the isolation. At all events, the procedure does not disrupt nuclei nor does it employ a chaotrope and the present invention is clearly distinguised therefrom.

The invention provides a method of determining a nucleic acid suspected of being present in cells containing nucleoproteins and nuclease, which method comprises lysing the cells in a lysis buffer comprising a chaotropic agent for disrupting nucleoproteins and which inhibits the nuclease, when DNA is being determined treating the lysate to single-strand it, contacting the lysate under hybridisation conditions with a polynucleotide having a nucleotide sequence complementary to a sequence present in the nucleic acid to be determined, and determining the extent of hybridisation.

Similarly, the invention also provides a method of isolating a nucleic acid present in cells containing nucleoproteins and nuclease, which method comprises lysing the cells in a lysis buffer comprising a chaotropic agent for disrupting nucleoproteins and which inhibits the nuclease, when DNA is to be isolated treating the lysate to single-strand it, contacting the lysate under hybridisation conditions with a polynucleotide having a nucleotide sequence complementary to a sequence present in the nucleic acid to be isolated and isolating the desired nucleic acid from the resultant hybrid.

The invention is applicable to a wide range of eukaryotic and prokaryotic cells, including protoplasts. In general, it is applicable to any cell which can be lysed by osmotic shock, enzymic treatment, or mechanical action such as homogenisation or disintegration in a bladed blender. It is therefore applicable to tissue culture animal cells, animal tissue, e.g. heart, liver or brain, homogenised in lysis buffer, blood cells, reticulocytes, lymphocytes, plant cells lysable by osmotic shock, and cells of bacteria, yeasts and other small microbial cells. Where the cell is lysable by osmotic shock the high molar concentration of salts present in the chaotrope will ordinarily bring about movement of salts into the cell sufficient to cause lysis. Where lysis is carried out by other means a buffer will be required and the chaotrope is incorporated in the buffer.

The chaotrope must dissociate nucleic acids from nucleoproteins and must inhibit nucleases. The preferred chaotropes are guanidinium salts in a concentration in the buffer at the time of hybridisation of at least 3 M, preferably about 4 M. Examples are guanidinium chloride and isothiocyanate. Other chaotropic agents are urea, lithium chloride and other isothiocyanates, in high molar concentrations. One preferred alternative to guanidinium salts is a mixture of 4 M lithium chloride and 8 M urea. Any of these chaotropes can include SDS to improve inhibition of nucleases.

The suggested upper limit of concentration of chaotrope is governed by a variety of factors. Where the hybridisation being performed involves two sequences which are respectively of the same nucleotide and its base-pairing partner, e.g. a poly(A) tail hybridised to a poly(T) or poly(U) sequence, it can be carried out in guanidinium salt of concentration 6 M or somewhat higher, although the solubility of the salts does not permit very much higher concentrations. Where the hybridisation involves sequences containing an assortment of different nucleotides, it is more difficult to carry out at reasonable temperatures, as explained hereinafter, and generally about 4.5 M guanidinium salt is preferably not exceeded. The concentrations of other chaotropes can be appropriately adjusted with the benefit of these guidelines for guanidinium salts.

The unexpectedness of the invention arises partly from the finding that the chaotrope permits hybridisation to take place at a relatively low temperature, despite its high concentration of salts. In other words the increase in disorder of hydrogen-bonded structures in the vicinity of the RNA appears to outweigh action of the salts in facilitating hybridisation.

The invention applies to the assay and isolation of all kinds of RNA and DNA. Since RNA exists partly in a single-stranded form in the cell it can be hybridised directly from the chaotrope-containing lysate without any special treatment. Obviously, the lysate must not be subjected to any treatment which would single-strand any DNA present, if one is seeking to assay or isolate the RNA alone, free of DNA. Where total RNA plus DNA is of interest the lysate can be heat-treated in a conventional way, typically by heating to $95^{\circ}C$ for 2 minutes to single-strand the DNA. Where it is desired to assay or isolate only the DNA, the RNA can be removed by treatment with alkali. This will normally denature the RNA into its component nucleotides and single-strand the DNA in one step. Such treatment is, of course, well known.

It is also desirable to reduce the molecular size of the DNA, e.g. to fragments 0.1 to 5 M-daltons to facilitate hybridisation. The size of the DNA fraction may be reduced e.g. by treatment of the lysate with ultrasound or by mechanical shear, for example by passing the lysate through a fine gauge hypodermic needle.

Many RNA molecules have a long "tail" of successive adenine residues at their 3'-end. These include RNA of polyadenylated viruses, e.g. in foot and mouth disease virus, and the RNA present in slime moulds. This so-called poly(A)$^+$-mRNA can be hybridised easily to a complementary poly(U) or poly(T) sequence, at temperatures from about $4^{\circ}C$ upwards.

In contrast, the optimum temperature for ordinary (untailed) DNA.RNA and DNA.DNA hybrid formation is normally regarded as $25^{\circ}C$ below the melting temperature $(T_m)$ of the bihelical product. At $T_m -25^{\circ}C$ hybridisation proceeds at an acceptable rate and the partly bihelical structure of RNA and denatured DNA is diminished and so is no longer a major barrier to hybrid formation. The temperature of the hybridisation reaction should therefore be kept low in order to minimise thermal hydrolysis of internucleotide bonds. To augment the action of the chaotrope in keeping the temperature required low, it is preferred to add an agent which will reduce this temperature. Formamide is a preferred such agent. Thus, lysis/formamide buffer (e.g. 4 M-guanidinium chloride etc./33% (v/v)-formamide) at temperatures within the range 35°C to 42°C was found to be especially suitable for RNA.DNA hybrid formation. In experimental practice, 1 volume of formamide was added to 2 volumes of lysate between 4.5 and 6 M in guanidinium salt.

While it is intended that the invention should be carried out on a crude lysate, minor mechanical treatments thereof are not to be considered excluded. Thus, for example, it might be desirable to clarify the lysate before hybridisation, in particular when the polynucleotide having the complementary sequence to the nucleic acid to be determined is insolubilised. The lysate may be clarified by slow speed centrifugation.

The hybridisation assay can be carried out by any method known per se or analogous to immunoassay methodology. It can be homogeneous or heterogeneous, conveniently heterogeneous, using an insolublised polynucleotide having a complementary nucleotide sequence to a sequence present in the nucleic acid to be determined. Preferred methods of assay are the sandwich type, as described by M. Virtanen et al., The Lancet, February 19, 1983, 381-383 and M. Ranki et al., Gene, 21, 77-85 (1983) and the competition or displacement assay. (A displacement assay is a quasi-competition assay in which the unlabelled partner is reacted first and the labelled partner is reacted subsequently, but is in excess relative to available binding sites so that not all of the labelled partner becomes bound.)

Thus, in one preferred embodiment of the invention, the extent of hybridisation is determined by a sandwich assay, in which the nucleic acid to be determined has first and second nucleotide sequences which are separate and mutually exclusive with respect to hybridisation, the lysate is contacted under hybridisation conditions with (1) a first polynucleotide which is insolublised and has a nucleotide sequence complementary to the first nucleotide sequence of the nucleic acid to be determined, and (2) a second polynucleotide which is in the liquid phase, labelled, and has a nucleotide sequence complementary to the second nucleotide sequence of the nucleic acid to be determined, the insoluble material is separated and the amount of label bound thereto is determined.

In another preferred embodiment of the invention, the extent of hybridisation is determined by a competition assay in which the lysate is contacted under hybridisation conditions with a first polynucleotide having a nucleotide sequence complementary to a sequence present in the nucleic acid to be determined and which is insolublised and the first polynucleotide is simultaneously or subsequently contacted under hybridisation conditions with a second polynucleotide which is in the liquid phase, labelled, and comprises a nucleotide sequence complementary to that of the said nucleotide sequence of the first polynucleotide, under conditions in which there is a molecular excess of first, insolublised polynucleotide with respect to each of the nucleic acid to be determined and the second, labelled polynucleotide but a molecular deficiency of the first polynucleotide over the sum of the nucleic acid to be determined and the second, labelled polynucleotide, the insoluble material is separated and the amount of label bound thereto or remaining in the liquid phase is determined.

It will be understood that the assay can be used for mere detection of the RNA or DNA, the extent of hybridisation being zero if none of that RNA or DNA is present in the cell.

Labelling is conveniently radiolabelling, but any other label can be used, for example biotin/avidin type labelling, e.g. as described in European Patent Specification 63879 (Yale University), chemiluminescent catalyst labelling, e.g. as mentioned in European Patent Specification 70687 (Standard Oil Company), enzyme labelling, e.g. as described by A.D.B. Malcolm et al., orally at the 604th Biochemical Society meeting, Cambridge, England on 1st July 1983 and fluorescent labelling, e.g. as described by M.S. Silver and A.R. Fersht, Biochemistry, 21, 6066-6072 (1982).

For isolation of the nucleic acid, only a single hybridisation step is required. Conveniently, the polynucleotide having the complementary sequence is insolublised (immobilised or supported or brought into or on a solid phase) and the bound nucleic acid is released from its column and from the support by any appropriate means well known in affinity chromatography of polynucleotides.

The kind of polynucleotide having the complementary sequence to the nucleic acid to be determined or isolated will depend on whether the nucleic acid has a poly(A) tail or other region in which there is a long continuous sequence of the same nucleotide (N). For hybridisation to a poly(A) sequence, alternative forms of affinity matrix which can be used include oligo(dT)-cellulose, poly(U) bound to cellulose, or to glass fibre discs, or poly(U)-diazobenzyloxymethyl paper and poly(U)-Al$^{3+}$-mica.

Where the nucleic acid lacks such a poly(N) sequence, the polynucleotide can be one which has previously been isolated incorporated in a vector such as a plasmid or phage and cloned. Alternatively, it can be a chemically synthesised oligomer, preferably of length at least about 18 or 19 nucleotides. The term polynucleotide includes any nucleic acid, polymer of nucleotide bases, oligomer, or the like which will hybridise to DNA or RNA as appropriate. The polynucleotide is preferably attached by covalent bonding to the support, which can be, for example, diazotised aminothiophenol paper. Nitrocellulose supports are less preferred.

The following Examples illustrate the invention.

General

All glassware was siliconised with dimethyldichlorosilane solution and autoclaved at 1 atmosphere gauge at 120°C for 15 minutes. All buffers and plasticware were also autoclaved as described above.

The term "lysis buffer", except where otherwise indicated, means, throughout the Examples, 6 M-guanidinium chloride/1 mM-beta-mercaptoethanol/15 mM-Tris-HCl, pH 7.5. "Lysis formamide buffer" means the lysis buffer containing 33% (v/v) of formamide.

- 10 -

EXAMPLE 1

A SINGLE-STEP PROCEDURE FOR THE ISOLATION OF THE POLY(A)$^+$-mRNA
FRACTION FROM CRUDE LYSATES OF PHYSARUM POLYCEPHALUM (SLIME MOULD) Buffers

Lysis buffer was either 6 M-guanidinium chloride/1 mM-
beta-mercaptoethanol/15 mM-Tris-HCl, pH 7.5, or 6 M-guanidinium
isothiocyanate/1 mM-beta-mercaptoethanol/10 mM-Tris-HCl, pH 7.5.

Eluting buffer for recovering poly(A)$^+$-mRNA.poly(U)-
Sepharose 4B was 90% (v/v) formamide/1 mM-EDTA/15 mM-Tris-HCl,
pH 7.5.

Wash buffer for purifying the poly(A)$^+$-mRNA.poly(U)-
Sepharose 4B complex was 4 M guanidinium chloride/1 mM-mercaptoethanol/15 mM-Tris-HCl, pH 7.5.

Storage buffer for poly(A)$^+$-mRNA was 10 mM-Tris-HCl, pH 7.0.
This buffer was also used to wash the poly(A)$^+$-mRNA.poly(U)-
Sepharose 4B complex immediately before treatment with eluting
buffer.

Preparation of poly(U)-Sepharose 4B

The dry gel supplied by Pharmacia AB was treated with 1 mM
NaCl for 5 minutes, washed with 0.1 M-NaCl (100 ml/g dry powder),
then with eluting buffer (25 ml/g dry powder) and finally
equilibrated with lysis buffer.

Growth of microplasmodia of Physarum polycephalum

Microplasmodia (strain M$_3$ C VIII) were grown at 26$^o$C in the
semi-defined medium described by J.W. Daniel and H.P. Rusch,
J. Gen. Microbiol. 25, 47-59 (1961),·except that chick embryo
extract was replaced by 1 ml of a haemetin solution (0.05% (w/v)
haemin in 1% (w/v) NaOH) for each 100 ml of medium. Microplasmodia
were grown in 125 ml of medium contained in 500 ml flasks which were
placed on a reciprocal shaker moving at a rate of 80 oscillations/
minute with a displacement of 10 cm/oscillation and subcultured
every 2 to 3 days to maintain log-phase growth. The microplasmodia
were harvested in log-phase approximately 2 days after inoculation.

Isolation of the poly(A)$^+$-mRNA fraction from crude lysates of microplasmodia of Physarum polycephalum

Microplasmodia were harvested by centrifuging the culture for 2 minutes at 4$^\circ$C in an MSE Mistral 4L centrifuge. The pelleted Physarum was weighed, cooled in an ice-bath, and lysis buffer (10 ml/g wet weight), pre-cooled to -15$^\circ$C, was added while the solution was stirred with a glass rod. The mixture was kept at -15$^\circ$C for approximately 20 minutes and the lysate was clarified either by low-speed centrifugation (10 minutes at 3000 rev/minute at 4$^\circ$C in an MSE Mistral 4L centrifuge) or at a higher speed, e.g. 10 minutes at 14,000 rev/minute at 4$^\circ$C in a Beckman J2-21 centrifuge. It is important, at this stage, to have removed all particulate matter which would contaminate the immobilised poly(U).

Poly(U)-Sepharose 4B (at least 50 mg dry weight/g wet weight of Physarum), equilibrated with lysis buffer, was then added to the lysate. 200 mg, 500 mg or 1 g of the gel were used according to the mass of Physarum being treated. The suspension was gently agitated to prevent the gel from settling, for 2 to 3 hours at 4$^\circ$C. The poly(U)-Separhose 4B was recovered by centrifugation (10 minutes at 3000 rev/minute). The pellet was resuspended in lysis buffer (10 ml) and centrifuged. This procedure was repeated until the supernatant was largely free of u.v. absorbing material ($A_{260}$ 0.01 to 0.1). The poly(U)-Sepharose 4B was then packed into a column and washed with lysis buffer, or with wash buffer, until the eluate was free from u.v. absorbing material ($A_{260}$ < 0.003). The column was then treated with storage buffer (15 ml). Bound poly(A)$^+$-mRNA fraction was recovered from the column by washing with eluting buffer. The poly(A)$^+$-mRNA fraction was recovered from the eluate by precipitation at -20$^\circ$C after the solution was made 1% in potassium acetate and 2.5 volumes of ethanol/volume of solution were added. The RNA was recovered by centrifugation in a Microfuge and dissolved in storage buffer. The procedure of precipitation and dissolution was repeated twice more, then the RNA was kept in storage buffer at -20$^\circ$C.

The procedure described above was found to be reliable, although not all the parameters were investigated to obtain optimal conditions.

The yield of purified poly(A)$^+$-mRNA was found to be approximately 25 micrograms poly(A)$^+$-mRNA fraction/g wet weight of microplasmodia. It is estimated that 1 g wet weight microplasmodia contains 40 micrograms poly(A)$^+$-mRNA and on this basis the yield of over 60% appears very reasonable.

Translation of poly(A)$^+$-mRNA in rabbit reticulocyte lysate

The assay was carried out as described by H.R.B. Pelham and R.J. Jackson, European Journal of Biochemistry 67 247-256 (1956). Nuclease-treated rabbit reticulocyte lysate (25 microlitres) containing 5 microcuries $^{35}$S-methionine and 19 non-radioactive amino acids was mixed with poly(A)$^+$-mRNA (up to 5 microlitres of mRNA in storage buffer) and the volume brought to 30 microlitres with the addition of water. Poly(A)$^+$-mRNA was tested at three different concentrations. The reaction mixtures were incubated at 34$^o$C for 90 minutes then cooled on ice. A sample (2 microlitres) was withdrawn from each assay, mixed with 0.1 M-KOH (150 microlitres), a drop of $H_2O_2$ was added, and samples were incubated at 34$^o$C for 10 minutes. An excess of ice-cold 10% (v/v) TCA was added and the samples were kept at 0$^o$C for 15 minutes to precipitate the protein. The precipitated protein was recovered by filtration using Oxoid cellulose acetate filters (Grade 0.45 micrometre). Assay tubes and filters were washed with 5% (v/v) TCA. Each filter was washed once in distilled water, then with ethanol, then with ethanol/diethyl ether (1:13 v/v), and finally with diethyl ether. Radioactivity of the filters was measured in a scintillation counter. The results, reported in Table 1 below, show that the slime mould poly(A)$^+$-mRNA was translated as efficiently as mouse poly(A)$^+$-mRNA isolated by a conventional procedure using phenol extractant.

## TABLE 1

| Species of mRNA added to lysate | Radioactivity of acid-insoluble protein (cpm) |
|---|---|
| None | 1,700 |
| 3T6 mouse mRNA (1 microgram) isolated by a conventional procedure | 13,400 |
| P. polycephalum poly(A)$^+$mRNA (0.5 microgram) | 9,060 |
| (1 microgram) | 12,600 |
| (2 micrograms) | 9,130 |

Separation of the products of cell-free synthesis

The radioactive proteins thus synthesised in the cell-free system were separated by electrophoresis through 1% (v/v) SDS-15% (w/v) polyacrylamide gels as described by U.K. Laemmli, Nature 227, 680-685 (1970). The gel was dried and the radioactive proteins were detected by fluorography. The molecular weight markers used were lysozyme (1400), trypsin inhibitor (19,000), creatine kinase (40,000), gluatamate dehydrogenase (53,000), catalase (60,000), fructose-6-phosphate kinase (81,000) and phosphorylase a (94,000). The radiolabelled proteins translated from the P. polycephalum poly(A)$^+$-mRNA ranged in size from 15,000 to 95,000 daltons.

Size distribution of poly(A) tails in several different mRNA fractions

The poly(A)$^+$-mRNA fraction was labelled at the 3'-end with [5-$^{32}$P]pCp using T4 RNA ligase. The radioactively labelled mRNA was separated on denaturing polyacrylamide gels. Six discrete bands of RNA within the size range 500 to 1800 nucleotides were cut out from the gel, the RNA was eluted and treated with RNAase T1 to release the poly(A) tail. The products were separated on 8% polyacrylamide sequencing gels. In each case the radioactivity revealed poly(A) tails ranging from approximately 15 nucleotides to 150 to 200 nucleotides. The size of the poly(A) tails of the P. polycephalum mRNA species which were examined is comparable with the size of the poly(A) tails of globin mRNA.

## EXAMPLE 2

Essentially the same method used in Example 1 was applied to liver tissue of the rat.

The liver of a rat was removed, part (3.5 g) was cut in small pieces, frozen in liquid nitrogen, and homogenised in lysis buffer (30 ml) using an Ultraturrax homogeniser. The homogenate was clarified at 14,000 rev/minute for 20 minutes in a Beckman J2-21 centrifuge. The supernatant was transferred to plastic centrifuge tube, poly(U)-Sepharose 4B (approximately 200 mg dry weight) prepared as Example 1 was added. The tube was gently agitated at $4^{\circ}$C for 2 hours. The poly(U)-Sepharose 4B was recovered by centrifugation, washed several times with lysis buffer at $4^{\circ}$C, packed into a disposable plastic column and again washed with lysis buffer. The column of poly(U)-Sepharose 4B was then washed at approximately $20^{\circ}$C with 5 ml of high salt buffer (0.5 M-NaCl/10 mM-EDTA/50% (v/v) formamide/Tris-HCl, pH 7.5) in the place of storage buffer. The poly(A)$^{+}$-mRNA was then eluted at approximately $20^{\circ}$C with the eluting buffer of Example 1. The poly(A)$^{+}$-mRNA fraction was precipitated by the addition of 0.01 volume of 3 M-potassium acetate, pH 7.0 and 2.5 volumes of ethanol after the solution had been kept at $-12^{\circ}$C for 2 hours. The yield was 150 micrograms poly(A)$^{+}$-mRNA. Its ability to direct protein synthesis in nuclease-treated rabbit reticulocyte lysates is illustrated in Table 2 below.

### TABLE 2

| Addition to nuclease-treated rabbit reticulocyte lysates | Radioactivity of $^{35}$S-labelled acid-insoluble protein (cpm) |
|---|---|
| None | 650 |
| Rat liver poly(A)$^{+}$-mRNA (2 micrograms) | 21,050 |
| Rat liver poly(A)$^{+}$-mRNA (4 micrograms) | 10,622 |

- 15 -

EXAMPLE 3

Essentially the same method used in Example 1 was applied to tissue of toad <u>Xenopus laevis,</u> and poly(A)$^+$-mRNA was recovered. In separate experiments the tissue used was (a) oocytes, and (b) liver.

Oocytes (3.7 g) were homogenised in (20 ml) lysis buffer (total volume 24 ml) cooled to $-12^{\circ}C$ using a glass homogeniser fitted with a PTFE plunger. Cell debris was removed by centrifuging for 20 minutes at 14,000 rev/minute in a Beckman J2-21 centrifuge. Poly(U)-Sepharose 4B prepared as in Example 1 was then added to the supernatant, as described in Example 2 and the poly(A)$^+$-mRNA fraction was then isolated essentially as described in Example 1.

A liver was removed from a toad, washed to remove blood and part (1 g) was added to lysis buffer (10 ml) cooled to $-12^{\circ}C$, cut up, then homogenised using a glass homogeniser and a mechanically driven PTFE plunger. The homogenate was filtered through butter muslin, the clarified by centrifugation at $4^{\circ}C$. Poly(U)-Sepharose 4B was then added and the poly(A)$^+$-mRNA fraction was isolated essentially as in Example 1. The yield was approximately 50 micrograms of poly (A)$^+$-mRNA.

The activity of the poly(A)$^+$-mRNA fractions in cell-free protein synthesis is given in Table 3 below.

TABLE 3

| Addition to nuclease-treated rabbit reticulocyte lysate | Radioactivity of $^{35}$S-labelled protein |
|---|---|
| None | 1,010 |
| Poly(A)$^+$-mRNA from oocytes (1.3 micrograms) | 8,800 |
| Poly(A)$^+$-mRNA from liver (2.1 micrograms) | 16,810 |

EXAMPLE 4

Essentially the same method used in Example 1 was applied to tissue of the rabbit and poly(A)$^+$-mRNA was recovered. In this

- 16 -

experiment the tissue was immature red blood cells (reticulocytes). Blood was obtained from anaemic rabbits. Washed, packed cells (10 ml) were resuspended at $0^{\circ}C$ in citrate/saline buffer (2 ml) and added with stirring to cooled $(-12^{\circ}C)$ lysis buffer (6 ml). The lysate was clarified by centrifuging for 20 minutes at 6,000 rev/minute in an MSE Mistral centrifuge. Poly(U)-Sepharose 4B prepared as in Example 1 was then added to the supernatant, as described in Example 2 and the poly(A)$^{+}$-mRNA fraction (160 micrograms) was isolated as in Example 1 and was shown to be active in mRNA directed protein biosynthesis (see Table 4).

### TABLE 4

| Additions to nuclease-treated rabbit reticulocyte lysate | Radioactivity of $^{35}S$-labelled acid-insoluble protein (cpm) |
|---|---|
| None | 810 |
| Poly(A)$^{+}$-mRNA | 11,200 |

### EXAMPLE 5

A SINGLE-STEP PROCEDURE FOR THE ISOLATION OF INDIVIDUAL mRNA SPECIES FROM CRUDE LYSATES OF PHYSARUM POLYCEPHALUM Isolation of p5-13 plasmid DNA

Escherichia coli strain FMA-10 carrying plasmid p5-13, which contains a 5 kbp Hind III-Bam H1 fragment of Physarum DNA inserted into plasmid pAC 184, was grown in the presence of chloramphenicol, and p5-13 plasmid DNA was isolated by the method of H.C. Birnboim and J. Doly, Nucleic Acids Research 7, 1513-1523 (1979).

Binding of DNA to aminothiophenol (ATP)-paper

ATP paper, which binds DNA covalently, was prepared according to a procedure developed by Seed, and quoted by P.F. Searle and J.R. Tata, Cell 23, 741-746 (1981). Sheets of Whatman 540 paper were shaken for 16 hours in a sealed bag with 350 mM-NaOH/30% (v/v) 1,4-butanediol diglyceryl ether, and 2 mg/ml of sodium borohydride. The paper was then washed for 1 hour with 0.25 M-NaOH/50% (v/v) ethanol, then incubated with shaking for 2 hours in 1 volume of 5% (w/v) 2-aminothiophenol in ethanol and 1 volume of 0.5 M-NaOH.

The paper was washed twice in absolute ethanol, and then with 0.1 M-HCl. The washing procedure was repeated twice more, and then the paper was washed in distilled water and dried in air, then stored in the dark at $20^{\circ}$C.

Immediately before use, ATP paper was diazotised by treatment with 1.2 M-HCl/ 0.03% (w/v)-NaNO$_2$ at $4^{\circ}$C for 30 minutes. The paper was washed twice with ice-cold distilled water, twice with ice-cold 50 mM-Na phosphate buffer, pH 6.5, and then was dried by blotting.

Plasmid DNA, made linear by treatment with restriction endonucleases _Bam_ Hl and _Hind_ III, was denatured by treatment with 80% (v/v)-dimethylsulphoxide/50 mM-Na phosphate, pH 6.5 at $80^{\circ}$C for 10 minutes, then cooled quickly on ice. Denatured (single-stranded) DNA (10 to 15 microlitres) was loaded onto a disc (7 mm diameter) of freshly diazotised ATP paper at $0^{\circ}$C, then left at $4^{\circ}$C for at least 4 hours to allow the DNA to bind. Before use, DNA/ATP-paper discs were washed in 80% (v/v) DMSO/50 mM-Na phosphate, pH 6.5 at $4^{\circ}$C for 30 minutes and in 0.1 x SSC at $95^{\circ}$C for 15 minutes to remove non-covalently bound DNA.

Isolation of p5-13 DNA-selected mRNA directly from crude cell lysates

Microplasmodia of _Physarum_ _polycephalum_ (10 g, 10 ml), grown as in Example 1, were lysed at $-20^{\circ}$C in 6 M guanidinium chloride lysis buffer (30 ml). The lysate (total volume 40 ml, 4.5 M in guanidinium chloride), was clarified by low-speed centrifugation, then formamide (20 ml) was added. The resulting lysis/formamide buffer was then 3 M in guanidinium chloride. Six discs of ATP paper, 1 cm in diameter, prepared as described above each carrying 10 micrograms of the immobilised p5-13 DNA were added to the lysis/formamide buffer solution, which was then gently shaken at $42^{\circ}$C for 40 hours. The paper discs were removed and washed at $42^{\circ}$C with lysis/formamide buffer (200 ml), then at $20^{\circ}$C with eluting buffer (2 ml). The bound mRNA was recovered by washing the discs with eluting buffer at $42^{\circ}$C. Yeast tRNA (25 micrograms) was added as carrier and RNA was precipitated at $-20^{\circ}$C by the addition of potassium acetate buffer (final concentration was 1%) and 2.5 volumes of ethanol. The RNA was redissovled in storage buffer and precipitated twice more, then redissolved in sterile distilled water (10 microlitres).

Separate tests were carried out in which poly(A)$^+$-mRNA from the P. polycephalum, labelled with $^{125}$I, was incubated at 37$^{o}$C and 42$^{o}$C and allowed to hybridise in lysis/formamide buffer, 4 M guanidinium chloride/33% (v/v) formamide, to p5-13 plasmid DNA. It was established beyond reasonable doubt that hybridisation had occurred, by observing a decrease in melting temperature of the produce when measured in increasing dilution of SSC and by its high resistance to RNAase. These tests indicated that under the similar conditions of cell lysis described above hybridisation must also have occurred.

Translation of mRNA in rabbit reticulocyte lysate

The assay was carried out as described for poly(A)$^+$-mRNA in Example 1. Poly(A)$^+$-mRNA isolated from mouse 3T6 cells was also assayed for comparison. The results are shown in Table 5 below.

TABLE 5

| Additions to nuclease-treated rabbit reticulocyte lysate | Radioactivity of $^{35}$S-labelled acid-insoluble protein (cpm) |
|---|---|
| None | 990 |
| poly(A)$^+$-mRNA isolated from mouse 3T6 cells (1 microgram), for comparison | 9,035 |
| p5-13 DNA-selected mRNA (1 microlitre) | 2,060 |
| p5-13 DNA-selected mRNA (3 microlitres) | 1,690 |
| p5-13 DNA-selected mRNA (5 microlitres) | 1,150 |

Separation of the products of cell-free synthesis

The radioactive proteins thus synthesised in the cell-free system were separated by electrophoresis through 1% (w/v) SDS-15% (w/v) polyacrylamide gels as described by U.K. Laemmli, Nature 227, 680-685 (1970). The gel was dried and the radioactive proteins were detected by fluorography. A protein of 25,000 $\pm$ 750 daltons was detected, but only in samples to which p5-13 DNA-selected-mRNA had been added. The minimum size of an mRNA species coding for a protein of this size is approximately 750 nucleotides excluding non-coding sequences.

EXAMPLE 6

COMPETITIVE ASSAYS OF rRNA AND rDNA IN LYSATES OF
PHYSARUM POLYCEPHALUM

Preparation of lysate

Microplasmodia of Physarum polycephalum, grown as in Example 1, were harvested, frozen in liquid nitrogen then lyophilised. The powder was stored at $-12^{\circ}$C. Normal lysate was prepared by adding 10 mg of the powder to 10 ml of lysis buffer kept at $-12^{\circ}$C. The lysate was further diluted with lysis buffer when necessary and stored at $-12^{\circ}$C.

Preparation of alkali-treated lysate

Lyophilised P. polycephalum powder (53 mg) was resuspended in lysis buffer (400 microlitres) at $-12^{\circ}$C. The solution was sonicated at $0^{\circ}$C by 4 x 14 second bursts of ultrasound. The solution was brought to approximately pH 12 by the addition of approximately 5 M-NaOH (75 microlitres) and kept at $42^{\circ}$C for 2.5 hours, cooled to $0^{\circ}$C and neutralised by the addition of approximately 5 M-HCl (50 microlitres), then stored at $-12^{\circ}$C.

Preparation of immobilised P. polycephalum DNA on ATP paper discs

Phage lambda 205 DNA containing a 14 kbp insert comprising genomic DNA for the whole of the 18S and 25S rRNA sequences of P. polycephalum was prepared by a standard procedure. This DNA was first diluted, typically twentyfold, by the addition of 80% (v/v) dimethyl sulphoxide/20 M-EDTA, pH 6.5 and heated to $90^{\circ}$C for 5 minutes, then cooled in ice. Samples were added to freshly diazotised ATP paper, diazotised as in Example 5, and left at $4^{\circ}$C overnight. Approximately 20% of the DNA was covalently bound to the paper. The discs were washed in 0.01 M-sodium phosphate buffer, pH 6.5, dried and then treated for 3 hours at $42^{\circ}$C with transfer RNA (10 mg in lysis/formamide buffer.

A calibration was carried out with purified rRNA obtained from _Physarum_ _polycephalum_, as follows. Small cylindrical plastic containers, 6 mm diameter x 23 mm, were used. Each pot contained 5 discs of ATP paper of 5 mm diameter prepared as described above, of which two were controls. To each pot were added lysis/formamide buffer (50 microlitres), containing $2.14 \times 10^4$ cpm of $^{125}$I rRNA probe (approximately $1 \times 10^4$ cpm/ng), transfer RNA (10 mg) and rRNA standard solution. The standard rRNA solution contained $1.1 \times 10^{-16}$ moles each of 18S rRNA and 25S rRNA per microlitre. The solution was kept at $42^{\circ}$C (with heating) for 42 hours.

The ATP discs were washed with lysis/formamide buffer (206 ml) for 30 minutes at $37^{\circ}$C and then with 0.1 x SSC (0.015 M-NaCl/ 0.0015 M-Na citrate, pH 7.0) for at least 2 hours.

Since this is a competition assay the relationship between the amount of radiolabel detected and the amount of rRNA is an inverse proportionality. When 1/R, where R is the counts per minute of radiation, was plotted against the volume V of the rRNA solution a straight line was obtained conforming to the equation $1/R = 8.4 \times 10^{-5} V + (1 \times 10^{-3})$. The averaged results were as shown in Table 6 below.

TABLE 6

| Volume (V) microlitres of standard _P. polycephalum_ rRNA solution (250 pg rRNA/ microlitre lysis buffer) | Radioactivity (R, cpm) | $\frac{1}{R} \times 10^3$ |
|---|---|---|
| 0 | 1022 | 0.98 |
| 1 | 890 | 1.12 |
| 2 | 835 | 1.20 |
| 4 | 742 | 1.35 |
| 8 | 603 | 1.66 |

The above assay was repeated, substituting for the standard rRNA solution (1) crude lysate of _P. polycephalum_, thereby to assay rRNA and (2) crude lysate of P. polycephalum treated with alkali to single-strand its DNA and denature its RNA, and thereby assay rDNA.

The results for the rRNA assay give a straight-line plot conforming to the equation $1/R = 2.03 \times 10^{-4} + (1 \times 10^{-3})$ and are shown in Table 7 below.

TABLE 7

| Volume (V) microlitres of P. polycephalum lysate containing 50 ng dry weight of P. polycephalum/microlitre lysis buffer | Radioactivity (R, cpm) | $\frac{1}{R} \times 10^3$ |
|---|---|---|
| 0 | 1010 | 0.99 |
| 2 | 814 | 1.23 |
| 4 | 578 | 1.73 |
| 8 | 403 | 2.48 |

Chemical analysis has revealed that 1 microgram of lyophilised P. polycephalum contains 30 ng total RNA of which 85% is known to be rRNA, i.e. 25 pg rRNA ($1.1 \times 10^{-17}$ moles)/ng P. polycephalum.

Thus, for example, 200 ng P. polycephalum powder is known to contain 25 x 200 = 5000 pg rRNA. The assay shows that 4 microlitres of lysate, containing the equivalent of 200 ng P. polycephalum powder, contains about $4 \times 250 \times \dfrac{2.03 \times 10^{-4}}{8.4 \times 10^{-5}}$ = 2400 pg rRNA, which is the right order of magnitude, and represents a high order of accuracy, considering the femtomole ($10^{-15}$ molar) concentrations employed and considering that the assay is probably not yet optimised.

The results for the rDNA assay give a straight-line plot conforming to the equation $1/R = 7.8 \times 10^{-5}V + (1 \times 10^{-3})$ and are shown in Table 8 below.

<div align="center">TABLE 8</div>

| Volume (V) microlitres of alkaline-treated P. polycephalum lysate containing 117 micrograms dry weight of P. polycephalum/ microlitre lysis buffer | Radioactivity (R, cpm) | $\frac{1}{R} \times 10^{-3}$ |
|---|---|---|
| 0 | 1010 | 0.99 |
| 2 (179 micrograms) | 1010 | 0.99 |
| 4 (350 micrograms) | 877 | 1.14 |
| 8 (684 micrograms) | 675 | 1.48 |

The volumes of alkaline-treated lysate were checked by weighing, weights being given in parenthesis.

From the known ratio of RNA to DNA (17:1) and the proportion of DNA that codes for rRNA (0.15% of DNA) it was estimated there were 2.6 pg ($1.2 \times 10^{-18}$ moles) of rDNA/microgram P. polycephalum powder. From the assay it is estimated that there are 2 pg rDNA/microgram P. polycephalum powder.

<div align="center">EXAMPLE 7</div>

SANDWICH ASSAY FOR POLYOMA VIRUS DNA

An assay for polyoma virus DNA middle-T antigen gene sequences is described. The ability of polyoma virus to transform cells and to induce tumours in rats results primarily from the action of the middle-T antigen. A new plasmid pAS101 was constructed, see B.A. Oostra, R. Harvey, B.K. Ely, A.F. Markham and A.E. Smith Nature 304, 456-459 (1983). These authors inserted a 2220 base-pair fragment of polyoma virus containing the origin of replication and the gene for the middle-T antigen into DNA of plasmid pAT153. These authors then showed that this new plasmid pAS101 transformed cells and induced tumours as a consequence of the integration of plasmid pAS101 DNA into the host genome.

An assay for pAS101 sequences was established as follows. The assay was based on three components: first, single-stranded pAT153 DNA immobilised e.g. on APT paper; secondly, $^{32}$P-labelled 2220 base-pair polyoma virus DNA fragment made single-stranded; and thirdly, the test sequence comprising pAT153 DNA and polyoma virus DNA made single-stranded. Upon hybridisation the radioactive label becomes associated with the immobilised DNA sequences since the nucleic acid species to be detected includes both pAT153 sequences and polyoma virus DNA sequences. Normal and transformed Rat-1 cells and pAS101-induced tumours were all readily lysed in lysis buffer.

Linearised, single-stranded pAT153 DNA (200 nanograms) was immobilised on ATP paper discs (7 mm diameter) and heated with transfer RNA, all as described in Example 5.

The radioactive probe was prepared by excising the polyoma virus DNA fragment from pAS101 by treatment with restriction endonucleases Eco R1 and Bam H1 and was purified by agarose gel electrophoresis. The separated 2220 base-pair fragment was $^{32}$P-labelled by nick translation. The probe was made single-stranded by heating to $90°C$ for 5 minutes before use. Approximately $2 \times 10^5$ cpm were used for each assay.

The standard solution (up to 4 nanolitres) of 25 pg/microlitre pAS101 in lysis buffer was made single-stranded by heat treatment at $90°C$ for 5 minutes before use. The final volume was 200 microlitres of lysis/formamide buffer. Hybridisation was for 18 hours at $42°C$. Each reaction mixture comprised 5 ATP paper discs, three of which had attached pAT153 DNA and two were controls.

TABLE 9

| Volume (microlitres) of single-stranded pAS101 DNA (25 pg/microlitre) | Radioactivity cpm |
|---|---|
| 0 | 220 |
| 1 | 793 |
| 2 | 1,490 |
| 4 | 2,457 |

Following the calibration done above, an assay was carried out to detect the polyoma DNA virus in transformed rat cells. Normal Rat-1 cells and Rat-1 cells transformed with pAS101 (rat-1-101 cells) were grown in tissue culture. The cells of one tissue culture plate were washed, lyophilised then dissolved in lysis buffer at $-12^{\circ}C$ (200 microlitres) and sonicated and treated with alkali essentially as described in Example 6. 1 volume of formamide per 2 volumes of lysate was then added and the solution was clarified in a Microfuge bench centrifuge. A sample of the supernatant (200 microlitres), radioactive probe ($2 \times 10^5$ cpm) and immobilised pAT153 DNA (200 ng loaded/disc) were used in an assay, as described above. The radioactivity incorporated was 720 cpm when lysate from transformed cells was used compared with 150 cpm when lysate from non-transformed Rat-1 cells was assayed.

43B

## CLAIMS

1.    A method of determining a nucleic acid suspected of being present in cells containing nucleoproteins and nuclease, which method comprises lysing the cells in a lysis buffer comprising a chaotropic agent for disrupting nucleoproteins and which inhibits the nuclease, when DNA is being determined treating the lysate to single-strand it, contacting the lysate under hybridisation conditions with a polynucleotide having a nucleotide sequence complementary to a sequence present in the nucleic acid to be determined, and determining the extent of hybridisation.

2.    A method according to Claim 1, wherein the chaotropic agent is a guanidinium salt and is present in the lysate in a concentration of at least 3 M at the time of hybridisation.

3.    A method according to Claim 1 or 2, wherein an agent which reduces the temperature required for hybridisation is added to the lysate before hybridisation.

4.    A method according to Claim 3, wherein the hybridisation-temperature reducing agent is formamide.

5.    A method according to any preceding claim, wherein the extent of hybridisation is determined by a sandwich assay, in which the nucleic acid to be determined has first and second nucleotide sequences which are separate and mutually exclusive with respect to hybridisation, the lysate is contacted under hybridisation conditions with (1) a first polynucleotide which is insolublised and has a nucleotide sequence complementary to the first nucleotide sequence of the nucleic acid to be determined, and (2) a second polynucleotide which is in the liquid phase, labelled, and has a nucleotide sequence complementary to the second nucleotide sequence of the nucleic acid to be determined, the insoluble material is separated and the amount of label bound thereto is determined.

6.    A method according to any one of Claims 1 to 4, wherein the extent of hybridisation is determined by a competition assay in which the lysate is contacted under hybridisation conditions with a first polynucleotide having a nucleotide sequence complementary to a sequence present in the nucleic acid to be determined and which is insolublised and the first polynucleotide is simultaneously or subsequently contacted under hybridisation conditions with a second polynucleotide which is in the liquid phase, labelled, and comprises a nucleotide sequence complementary to that of the said nucleotide sequence of the first polynucleotide, under conditions in which there is a molecular excess of first, insolublised polynucleotide with respect to each of the nucleic acid to be determined and the second, labelled polynucleotide but a molecular deficiency of the first polynucleotide over the sum of the nucleic acid to be determined and the second, labelled polynucleotide, the insoluble material is separated and the amount of label bound thereto or remaining in the liquid phase is determined.

7. A method of isolating a nucleic acid present in cells containing nucleoproteins and nuclease, which method comprises lysing the cells in a lysis buffer comprising a chaotropic agent for disrupting nucleoproteins and which inhibits the nuclease, when DNA is to be isolated treating the lysate to single-strand it, contacting the lysate under hybridisation conditions with a polynucleotide having a nucleotide sequence complementary to a sequence present in the nucleic acid to be isolated and isolating the desired nucleic acid from the resultant hybrid.

43B

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | CHEMICAL ABSTRACTS, vol. 77, no. 3, July 17, 1972, page 227, no. 16094j, Columbus, Ohio, US; J.F. SCOTT et al.: "Isolation of ribonucleic acid from animal tissue by use of chaotropic agents" & ANAL. BIOCHEM. 1972, 47(2), 471-80 * Whole abstract * | 1-5,7 | C 12 Q 1/68 |
| X,Y | CHEMICAL ABSTRACTS, vol. 74, no. 9, March 1, 1971, page 9, no. 38292n, Columbus, Ohio, US; P. KOURILSKY et al.: "DNA-RNA hybridization at low temperature in the presence of urea" & BIOCHEM. BIOPHYS. RES. COMMUN. 1970, 41(4), 1080-7 * Whole abstract * | 1-5,7 | |
| | | | TECHNICAL FIELDS SEARCHED (Int. Cl. ³) |
| X,Y | CHEMICAL ABSTRACTS, vol. 77, no. 5, July 31, 1972, page 246, no. 31241y, Columbus, Ohio, US; PH. KOURILSKY et al.: "DNA-DNA hybridization on filters at low temperature in the presence of formamide or urea" & BIOCHIMIE 1971, 53(10), 1111-14 * Whole abstract * | 1-5,7 | C 12 Q G 01 N |

--- -/-

The present search report has been drawn up for all claims

| Place of search THE HAGUE | Date of completion of the search 20-08-1984 | Examiner OSBORNE H.H. |
|---|---|---|

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. ⁸) |
|---|---|---|---|
| Y | CHEMICAL ABSTRACTS, vol. 85, no. 9, August 30, 1976, page 212, no. 58652k, Columbus, Ohio, US; C. MUSOLAN et al.: "Effect of urea on intracellular ribonuclease from Aspergillus niger" & REV. ROUM. BIOCHIM. 1975, 12(4), 227-32 * Whole Abstract * | 1 | |
| D,Y | GENE, vol. 21, 1983, pages 77-85, Elsevier Biomedical Press; M. RANKI et al.: "Sandwich hybridization as a convenient method for the detection of nucleic acids in crude samples" * Whole article * | 1-5,7 | |
| A | US-A-4 235 960 (E. SASSE & D. YORDE) * Abstract; examples 1-2 * | 6 | TECHNICAL FIELDS SEARCHED (Int. Cl. ³) |
| A | GB-A-2 019 408 (INSTITUT PASTEUR) * Whole document * | 1-7 | |
| A | US-A-4 358 535 (S. FALKOW & S. MOSELEY) * Whole document * | 1-7 | |

The present search report has been drawn up for all claims

| Place of search | Date of completion of the search | Examiner |
|---|---|---|
| THE HAGUE | 20-08-1984 | OSBORNE H.H. |